# EUROPEAN PATENT SPECIFICATION

(11) **EP 3 082 882 B1**
(45) Date of publication and mention of the grant of the patent: **04.04.2018**
(21) Application number: 14838814.3
(22) Date of filing: 11.12.2014
(51) Int. Cl.: A61L 2/10, C02F 1/32, E03D 9/00, C02F 103/00, E03D 11/18

(54) **UV BACTERIA-KILLING DEVICE FOR WATER CLOSET SIPHON HOUSED IN CYLINDRICAL SHAPED CONTAINER**
UV-BAKTERIENABTÖTUNGSVORRICHTUNG FÜR WC-SIPHON IN EINEM ZYLINDRISCH GEFORMTEN BEHÄLTER
DISPOSITIF UV TUEUR DE BACTÉRIES PRÉSENTES DANS LES SIPHONS DES TOILETTES

(30) Priority: 17.12.2013 IT VT20130009
(43) Date of publication of application: 26.10.2016
(73) Proprietor: Vitali, Gastone, 01033 Civita Castellana (VT) (IT)
(72) Inventor: Vitali, Gastone, 01033 Civita Castellana (VT) (IT)
(74) Representative: Garavelli, Paolo
(86) International application number: PCT/IT2014/000328
(87) International publication number: WO 2015/092822

(56) References cited:
- WO-A1-2011/092544
- US-A- 5 891 329
- US-B1- 6 685 890

## Description

The invention is set out in the appended set of claims. Though there are various types of bacteria-killing devies patented by the Applicant of the present Application, no universal bacteria-killing devices are known in the art, of the below described type.

US-A-5 891 329, US-B1-6 685 890 and WO-A1-2011 / 092544 disclose a system according to the preamble of Claim 1.

Object of the present invention is solving the above prior art problems, by providing a system, whose technical features, if applied to the syphon of the water closet, are those of avoiding the return of infectious agents, such as viruses and bacteria, from sewages to the discharging elements of the water closet and the diffusion on the whole surface of the water closet.

The above and other objects and advantages of the invention, as will appear from the following description, are obtained with a system comprising a water closet and a bacteria-killing device as claimed in Claim 1.

It is intended that the enclosed claims are an integral part of the present description.

The present invention will be better described by some preferred embodiments thereof, provided as a non-limiting example, with reference to the enclosed drawings, in which:
- Figure 1 is a front view of a water closet to which the bacteria-killing device of the present invention is applied;
- Figure 2 is a block diagram of a UV (Ultra Violet) lamp and of its connections as used in the bacteria-killing device of the present invention;
- Figure 3 is a side sectional view of the water closet of Figure 1; and
- Figure 4 is a rear sectional view of the water closet of Figure 1 or 2 having installed therein the bacteria-killing device of the present invention.

With reference to the Figures, a preferred embodiment of the bacteria-killing device of the present invention is shown and described. It will be immediately obvious that numerous variations and modifications (for example related to shape, sizes, various colors and parts with equivalent functionalities) could be made within the scope of the enclosed claims.

With reference to the drawings, the present invention is related to a system comprising a water closet and a UV (Ultra Violet) bacteria-killing system for the syphon of all series of water closets 8.

In the bathroom, in particular in public places such as hospitals, hotels but also residential homes, the problem of disinfection and hygiene is particularly felt, also due to standards in force related to the control of viruses and bacteria.

Subject matter of the invention is a universal application of the system to all series of water closets 8.

The system consists of a UV lamp 1 applied on the side of the water closet ceramics through a cylinder 2, preferably made of polycarbonate, containing the germ-killing UV lamp 1 which irradiates water contained in the syphon to remove viruses and bacteria coming from sewages.

It can be applied to all series of water closets (on the ground, suspended, for disabled people, etc.) and for new types of productions.

For a better understanding of the invention, the application modes of the UV lamp 1 are included below.

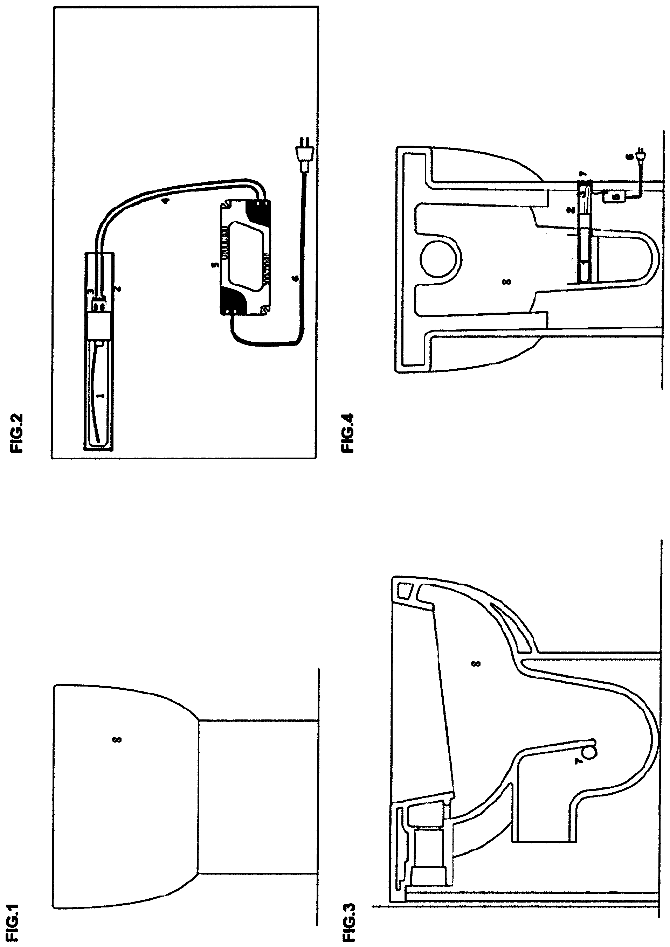

Figure 4 is a rear and side view of a normal water closet 8 with the lateral insertion of the UV lamp 1 contained inside a cylinder 2 made of polycarbonate placed inside the water closed 8.

The UV lamp 1 is supplied by a small box containing a switching-on device and a 12/24V transformer 5 through a suitable wiring 3 and a wire 4 for connection to the UV lamp 1. The box with transformer 5 is supplied through a connection plug 6 to the external mains.

As can be seen in Figures 3 and 4, the lamp 1, adequately protected and inserted into the cylinder 2 together with the wiring 3, is placed in a hole 7 suitably obtained in the water closet 8, in order to remain completely immersed in the discharge water, and be placed (Figure 3) in the syphon in order to intercept all viruses and bacteria returning from sewages.

The UV lamp 1 remains always on (24 hours on 24) for a complete and efficient removal of viruses and bacteria.

Summarizing, the system comprising a water closet and a bacteria-killing device for water closets 8 of the invention is adapted to remove viruses and bacteria coming from sewages and which can be found inside the water closet 8, and comprises:
- a container 2 containing one or more UV lamps 1 and placed inside a hole 7 obtained in the water closet 8 so that the container 2 is completely immersed in the discharge water of the syphon of the water closet 8 in order to intercept all viruses and bacteria returning from sewages; and
- a small box containing a switching-on device and a 12/24V transformer 5 connected to the container 2 and which allows switching on the UV lamp 1 in the syphon of the water closet 8.

The container 2 is of an elongated cylindrical shape and is adapted to be inserted in the hole 7 obtained on one of the sides of the water closet 8 in order to hermetically close the hole 7.

## Claims

1. System comprising a water closet (8) and a bacteria-killing device adapted to remove viruses and bacteria coming from sewages and which can be found inside the water closet (8), wherein said bacteria-killing device comprises:
- a container (2) containing one or more UV lamps (1) and placed inside a hole (7) obtained in the water closet (8) so that the container (2) is immersible in the discharge water of the syphon of the water closet (8) in order to intercept all viruses and bacteria returning from sewages; and
- a small box containing a switching-on device and a 12/24V transformer (5) connected to the container (2) and which allows switching on the UV lamp (1) in the syphon of the water closet (8);
**characterized in that** said container (2) is of an elongated cylindrical shape and is adapted to be inserted in said hole (7) obtained on one of the sides of said water closet (8) in order to hermetically close said hole (7).

2. Use of a bacteria-killing device for a water closet (8) in order to remove viruses and bacteria coming from sewages and which can be found inside the water closet (8), wherein said bacteria-killing device comprises:
- a container (2) containing one or more UV lamps (1) and placed inside a hole (7) obtained in the water closet (8) so that the container (2) is immersible in the discharge water of the syphon of the water closet (8) in order to intercept all viruses and bacteria returning from sewages; and
- a small box containing a switching-on device and a 12/24V transformer (5) connected to the container (2) and which allows switching on the UV lamp (1) in the syphon of the water closet (8);
**characterized in that** said container (2) is of an elongated cylindrical shape and is adapted to be inserted in said hole (7) obtained on one of the sides of said water closet (8) in order to hermetically close said hole (7).

## Patentansprüche

1. System, das ein Wasserklosett (8) und einen Sterilisator enthält, der dazu dient, Viren und Bakterien zu entfernen, die aus dem Kanalnetz kommen und die sich im Wasserklosett (8) befinden, in dem der genannte Sterilisator Folgendes enthält:
- einen Behälter (2), der eine oder mehrere UV-Lampen (1) enthält und der in einer Öffnung (7) angebracht wird, die im Wasserklosett angefertigt (8) wurde, sodass der Behälter (2) in das Abwasser des Siphons des Wasserklosetts (8) getaucht werden kann, um alle Viren und Bakterien abzufangen, die aus dem Kanalnetz zurückkehren; und
- eine kleine Schachtel, die einen Anzünder und einen 12/24v Transformator (5) enthält, der mit dem Behälter (2) verbunden ist und die Einschaltung der UV-Lampe (1) im Siphon des Wasserklosetts (8) ermöglicht;
und **dadurch gekennzeichnet ist, dass** der genannte Behälter (2) eine längliche Zylinderform hat und dazu dient, in die genannte Öffnung (7) eingeführt zu werden, die auf einer der Seiten des genannten Wasserklosetts (8) angebracht wurde, um die genannte Öffnung (7) hermetisch zu schließen.

2. Verwendung eines Sterilisators für ein Wasserklosett (8), um Viren und Bakterien zu entfernen, die aus dem Kanalnetz kommen und die sich im Wasserklosett (8) befinden, in dem der genannte Sterilisator Folgendes enthält:
- einen Behälter (2), der eine oder mehrere UV-Lampen (1) enthält und der in einer Öffnung (7) angebracht wird, die im Wasserklosett angefertigt (8) wurde, sodass der Behälter (2) in das Abwasser des Siphons des Wasserklosetts (8) getaucht werden kann, um alle Viren und Bakterien zu abzufangen, die aus dem Kanalnetz zurückkehren; und
- eine kleine Schachtel, die einen Anzünder und einen 12/24v Transformator (5) enthält, der mit dem Behälter (2) verbunden ist und die Einschaltung der UV-Lampe (1) im Siphon des Wasserklosetts (8) ermöglicht;
und **dadurch gekennzeichnet ist, dass** der genannte Behälter (2) eine längliche Zylinderform hat und dazu dient, in die genannte Öffnung (7) eingeführt zu werden, die auf einer der Seiten des genannten Wasserklosetts (8) angebracht wurde, um die genannte Öffnung (7) hermetisch zu schließen.

## Revendications

1. Système comprenant un WC (8) et un stérilisateur apte à éliminer les virus et les bactéries provenant des égouts qui se trouvent à l'intérieur du WC (8), où le stérilisateur comprend :
- un conteneur (2), renfermant une ou plusieurs lampes UV (1), placé à l'intérieur d'un trou (7) creusé dans le WC (8) de sorte à être plongé dans l'eau d'évacuation du siphon du WC (8) afin d'intercepter tous les virus et les bactéries remontant les égouts ; et
- un boîtier contenant un allumeur et transformateur à 12/24v (5) relié au conteneur (2) qui permet d'allumer la lampe UV (1) dans le siphon du WC (8) ;
**caractérisé en ce que** le conteneur (2) est de forme cylindrique allongée et est apte à être inséré dans le trou (7) creusé sur l'un des côtés du WC (8) de sorte à fermer hermétiquement le trou (7).

2. Utilisation d'un stérilisateur pour WC (8) servant à éliminer les virus et les bactéries provenant des égouts et qui se trouvent à l'intérieur du WC (8) ; où le stérilisateur comprend :
- un conteneur (2), renfermant une ou plusieurs lampes UV (1), placé à l'intérieur d'un trou (7) creusé dans le WC (8) de sorte à être plongé dans l'eau d'évacuation du siphon du WC (8) afin d'intercepter tous les virus et les bactéries remontant les égouts ; et
- un boîtier contenant un allumeur et transformateur à 12/24v (5) relié au conteneur (2) qui permet d'allumer la lampe UV (1) dans le siphon du WC (8) ;
**caractérisé en ce que** le conteneur (2) est de forme cylindrique et est apte à être inséré dans le trou (7) creusé sur l'un des côtés du WC (8) de sorte à fermer hermétiquement le trou (7).
